# EUROPEAN PATENT APPLICATION

(11) **EP 0 952 209 A2**
(43) Date of publication of application: **27.10.1999**
(21) Application number: 99301235.0
(22) Date of filing: 19.02.1999
(51) Int. Cl.: C12M 1/24, C12Q 1/10

(54) **Device and method for differentiation and identification of enterobacteria**

(30) Priority: 19.02.1998 IL 12335998
(71) Applicant: NOVAMED LTD., 91531 Jerusalem (IL)
(72) Inventor: Shmilovitz, Moshe, Haifa (IL)
(74) Representative: Silverman, Warren

(57) **Abstract**

A single test tube device contains at least three reactive components for differentiation and identification of enterobacteria, in a test tube (2), namely a first semisolid agar medium (4), a second solid agar medium (3) on top of first agar and a cap (7) containing agar medium (8).

## Description

This invention relates to a novel single tube device for multi-reactive components for use in particular for the differentiation and identification of enterobacteria.

A method and device for differentiation and identification of enterobacteria is described in IL patent No. 55720, by Moshe Shmilovitz. In accordance with this patent, there is provided a two component single tube containing a butt of semisolid modified urea agar at the bottom of the tube, a slant above this and above the slant a butt of modified Kligler iron agar. Firstly, the tube is closed off by a lignin plug. A filter paper strip impregnated with indole reagent is provided in an additional tube. This impregnated filter strip is to be attached to the open end of the tube. In accordance with this patent, a method for differentiation between and identification of enterobacteria is described, in which bacterial cultures or isolates are inoculated, into the two component media, an Indole detection filter paper is attached by folding the paper strip partly outside and partly inside the tube at the upper end, so as not to touch the agar, the cap is replaced so as to loosely close the tube, and the tube in incubated. In order to differentiate for example between E.coli and citrobater diversus, there is need for an additional culture medium (citrate medium) in an additional tube or plate.

This system has some drawbacks. The test protocol according to the above patent requires a combination of an available two component medium tube and an additional filter paper which has to be inserted after inoculation. This is troublesome, interferes with the application of the cap, and sometimes the paper is torn. Another drawback of the previous art is the need to use an additional citrate medium plate, which may lead to patient identification being lost.

It is therefore an object of the present invention to have more of the components needed incorporated in a single tube to simplify the handling, to save incubator place and to prevent loss of identification of patients.

In accordance with the invention, there is provided a single test tube device containing at least three reactive components for differentiation and identification of enterobacteria the device comprising: a test tube containing a first semisolid agar medium, a second solid agar medium on top of the first agar medium, and a cap containing an agar medium.

With one form of tube device embodying the invention (Embodiment la), there is provided a three reactive component test tube comprising: a semisolid modified urea agar - (formulation given in IL patent No. 55720) as a first phase, placed at the bottom of the tube; a second phase of a modified Kligler iron agar - (formulation given in IL patent No. 55720) is placed on top of the first phase, and an upper cap in which citrate medium is placed. A lignin plug, pre-impregnated with indole reagent is provided in separate packaging. This plug can be inserted into the tube in a simple and trouble-free manner before replacement of the cap. In operation, the cap is removed from the tube, the two agar phases are inoculated with a sample as described earlier, the plug with the indole reagent is placed in the tube, the citrate agar in the cap is also inoculated with the sample, and the cap is then loosely placed in the mouth of the tube on top of the plug. The tube is then incubated.

In accordance with another form of tube embodying the invention (Embodiment 2a), there is provided a four reactive component test tube comprising: a semisolid modified urea agar - as a first phase, placed at the bottom of the tube; a second phase of a modified Kligler iron agar - (formulation given in IL patent No. 55720) placed on top of the first phase; a lignin plug pre-impregnated with indole reagent inserted in the tube and partly occupying the remaining space in the tube, and an upper cap containing citrate agar medium closing the tube and covering the plug. In operation, the cap and the plug are removed from the tube, the two phases are inoculated with a sample as described earlier, the plug with the indole reagent is replaced in the tube, the citrate agar in the cap is also inoculated with the sample, and the cap is then loosely placed on top of the tube over the plug. The tube is then incubated.

In accordance with another form of tube embodying the invention (Embodiment 1b) there is provided a three reactive component test tube comprising: a semisolid modified urea agar - as a first phase, placed at the bottom of the tube, a second phase of a modified Kligler iron agar - placed on top of the first phase; and an upper cap incorporating a central tube extending from the bottom of the cap and surrounded by citrate medium. In operation, the cap is removed from the tube, the agar phases in the tube are inoculated with a sample as described earlier and a rod with Indole reagent is inserted into the tube in the cap. The citrate agar in the cap is also inoculated with the sample. The cap is then loosely placed on top of the tube and the tube is incubated.

In accordance with yet another form of tube embodying the invention (Embodiment 2b), there is provided a four reactive component test tube comprising: a semisolid modified urea agar - as a first phase, placed at the bottom of the tube; a second phase of a modified Kligler iron agar placed on top of the first phase; a rod or tube containing a medium pre-impregnated with Indole reagent attached to a cap; and the cap itself containing citrate medium. In operation, the cap is removed from the tube, the two agar phases in the tube are inoculated with a sample as described earlier, the citrate agar in the cap is also inoculated with the sample, and then the cap is loosely placed on top of the tube. Finally the tube is incubated.

For a better understanding of the invention, and to show how the same may be carried into effect, reference will now be made, by way of example, to the accompanying drawings, in which:-
Fig. 1 illustrates two embodiments of the invention, with
   Fig. 1a being a perspective view of a three reactive ingredient assembled device;
   Fig. 1b being a perspective view of a pre-impregnated lignin plug; and
   Figs. 1c-1, c-2, c-3 being perspective views of the components of the device, prior to incubation;
   and Fig. 1d being a perspective view of a four reactive ingredient assembled device;
   Fig. 1e demonstrating the inoculation of the two media tube; and
   Fig. 1f showing the inoculation of citrate media in the cap,
   while, in addition,
   Fig. 1g is a perspective view of an empty cap;
   Fig. 1h is a lengthwise cross-sectional view of the empty cap;
   Fig. 1i is a like cross-sectional view of the cap, whose recess is filled with reaction medium;
   Fig. 1j is a transverse cross-sectional view of the empty cap at one end thereof; and
   Fig. 1k is a like cross-sectional view of the cap when its end is filled with reaction medium.
Fig. 2 illustrates a third embodiment of the invention, wherein:
   Fig. 2a is a cross-sectional view of an assembled three active ingredient tube device;
   Fig. 2b shows a longitudinal cross-sectional view of the components of the device;
   Fig. 2c is a longitudinal cross-sectional view of the cap and inserted rod when assembled together;
   Fig. 2d is a longitudinal cross-sectional view of a pre-impregnated rod;
   Fig. 2e shows the inoculation of the citrate medium in the cap; and
   Fig. 2f is a longitudinal cross-sectional view of the assembled tube.
Fig. 3 illustrates a fourth embodiment of the invention, wherein:
   Fig. 3a is a longitudinal cross-sectional view of the assembled tube;
   Fig. 3b shows a longitudinal cross-sectional view of the components of the device;
   Fig. 3c is a transverse cross-sectional view of the empty cap at one end thereof; and
   Fig. 3d is a like cross-sectional view of the cap filled with reaction medium.

Common to all embodiments of the present invention is a container 20 comprising a test tube 2 having an upper open end 9 and containing semisolid modified urea agar 4 at the bottom of the tube and modified Kligler agar 3 placed on top of urea agar 4, the Kligler agar having a slant upper face 5.

In accordance with Embodiment la, the device also comprises: a cover member 10 comprising a cap 7 containing citrate agar 8 at the upper-inside end (when viewed in the assembled orientation), which cap has an open end 14 and a closed end 15, and a recess 12 at the in the closed end to accommodate the citrate medium 8, which recess 12 has wall 11 leaving space 13 for the accommodation of the wall of the test tube so as to achieve hermetic sealing. An impregnated member 30 comprising a lignin plug 1 having an indole reagent impregnated zone 6 is provided separately.

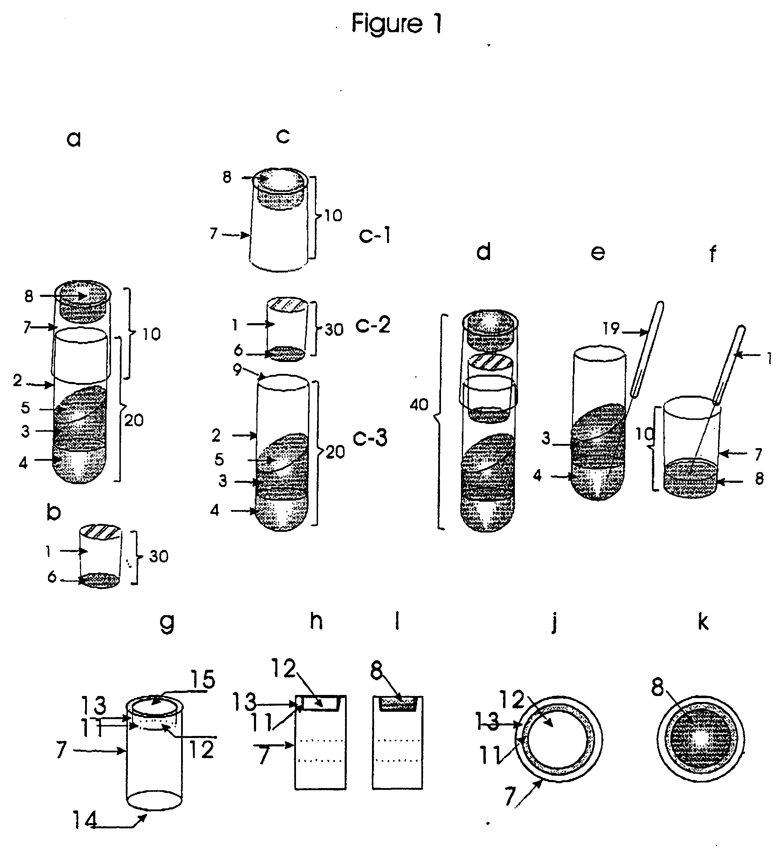

In operation, the cover member 10 is first removed, the sample to be tested is inoculated by an inoculation member 19 inserted into the two media test tube 20 as shown in Fig. le and into the citrate agar - in the cover member 10 - as shown in Fig. 1f. The impregnated member 30 is placed in the tube so that part of the plug extends inside the tube, but not as far as the agar, and part of the plug is outside the tube. Cover member 10 is then loosely replaced on the tube, and the assembled device is placed in an incubator. The results of the test are analyzed similarly to the protocol given in patent IL 55720.

In accordance with Embodiment 2a, the device of the Embodiment la, is modified to have impregnated member 30, which comprises a lignin plug 1 having an indole reagent impregnated zone 6 incorporated therein from the outset. This impregnated member is pre-inserted into the tube 20 and the cover member 30 is placed on top of the impregnated member and the test tube. In operation, the cover member 10 and the impregnated member 30 are first removed. The rest of the protocol is similar to that described in connection with Embodiment 1a.

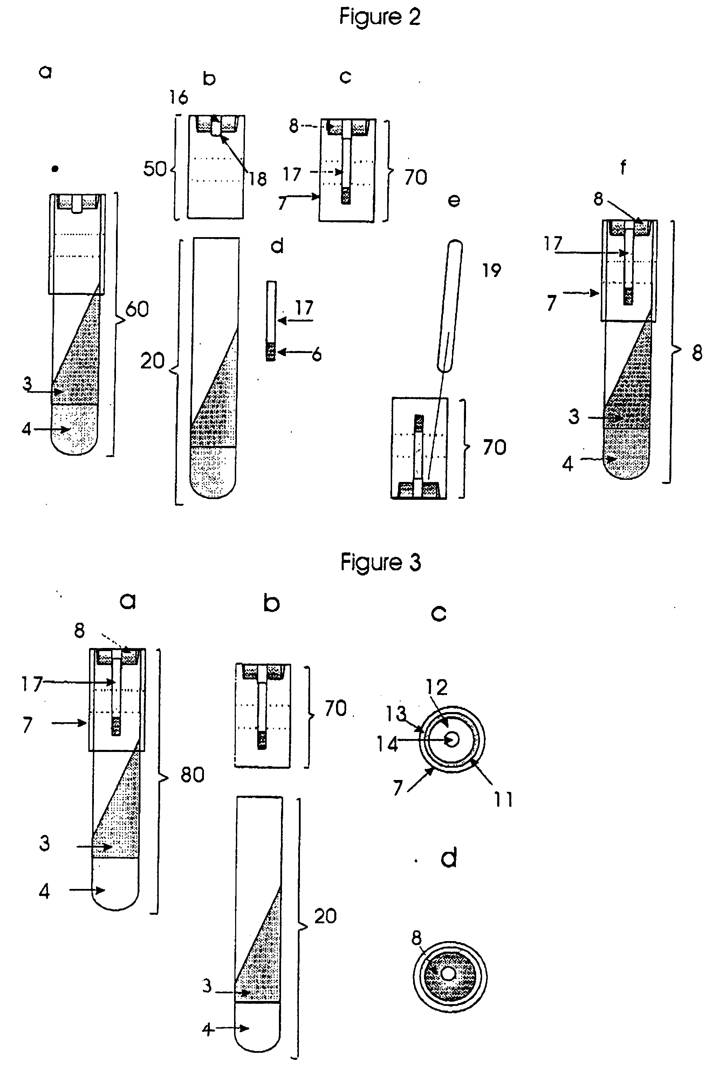

Embodiment 1b is similar to Embodiment la, except that cover member 30 has an additional small tube 16 (Fig. 2b) fused or welded to the bottom of the cover member at one end so as to be open at end 18 towards the open end of the cover member. This open end 18 of tube 16 is sized to accommodate rod 17 (Fig. 2d) which rod has an impregnated lower zone 6. This form of cover member is denoted by 50 in Fig. 2b. In this cover member 50, the citrate agar will occupy the space 12 which is defined between the walls 11 and the walls 14 of tube 16 as shown in Fig. 3c and Fig. 3d. In operation, the cover member 50 is first removed, then the impregnated rod is attached to the tube 16 of the cover member 50. This assembly of cover member 50 and rod 17 is denoted by 70 in Fig. 2c. The rest of the protocol is similar to that described in connection with Embodiment 1a.

Embodiment 2b is similar to Embodiment 1b, except that cover member 50 has, in addition a pre-inserted rod 17 with a pre-impregnated zone 6. This is denoted by 70 in Fig. 3b. In operation, only the cover member 70 must be removed, as the rod is attached to the cover member, and no handling of the rod is involved in this embodiment. The rest of the protocol is similar to that described in connection with Embodiment 1a.

## Claims

1. A single test tube device containing at least three reactive components for differentiation and identification of enterobacteria the device comprising:
a test tube (2) containing a first semisolid agar medium (4), a second solid agar medium (3) on top of the first agar medium, and a cap (7) containing agar medium (8).

2. A device as claimed in claim 1 having an additional reactive component (6) impregnated into an absorbent material (1) placed in an intermediate position with respect to the agar medium (3) in the tube (2) and the agar medium (8) at the cap.

3. A device as claimed in claim 1 or 2, wherein the two media in the test tube (2) are a semisolid modified urea agar (4) at the bottom of the tube, a slant modified Kligler iron agar (3) on top of the urea agar and wherein citrate agar (8) is present at the bottom of the cap (7), the device being associated as a first component with indole reagent impregnation material (6) as a second component either within the test tube or packaged separately for positioning in the tube when the tube is in use.

4. A device as claimed in any of claims 1 to 3, wherein the cap (7) has a recess (12) accommodating agar medium (8), which recess is at the inside of the closed bottom (15) of said cap and is spaced apart from the wall of the cap forming an annular space (13) to accommodate open end (9) of the tube, so as to achieve hermetic sealing.

5. A device as claimed in claim 4 where an additional tubular member (16) extends from the bottom (15) of the cap (7), toward open end (14) of the cap, which tubular member is capable of receiving a rod member (17) impregnated with indole reagent.

6. A device as claimed in claim 5 wherein a rod member (17) having an Indole reagent - impregnated zone (6) is inserted into the tubular member (16) to form a combined cap and rod.

7. A method for differentiation and identification of enterobacteria wherein, using a tube device as defined in claim 1, the cap is removed from the tube, the two media in the tube and the medium in the cap are inoculated, an additional reactive component-impregnated plug is placed in the upper part of the tube, the cap is loosely placed on the tube above the plug and the tube device is incubated for an appropriate time and temperature.

8. A method for differentiation and identification of enterobacteria wherein, using a tube device as defined in claim 2, the cap and a text reagent-impregnated plug, the latter being initially within the tube, are removed from the tube, the two media in the tube and the medium in the cap are inoculated, the plug is replaced in the upper part of the tube, the cap is loosely replaced on top of the plug and tube and the tube device is incubated for an appropriate time and temperature.

9. A method for differentiation and identification of enterobacteria wherein, using a tube device as defined in claim 5, the cap is removed from the tube, the two media in the tube and the medium in the cap are inoculated, the impregnated rod is inserted into the tubular member comprised by the cap, the cap is placed loosely on the tube and the tube device is incubated for an appropriate time and temperature.

10. A method for differentiation and identification of enterobacteria wherein, using a tube device as defined in claim 2 or 6, the cap containing citrate agar and an indole reagent impregnated rod being attached to the cap, in which method the cap with the impregnated rod is removed, the two media in the tube and the medium in the cap are inoculated, the cap is loosely replaced on the tube and the tube device is incubated for an appropriate time and temperature.
